(19) Europäisches Patentamt
European Patent Office
Office européen des brevets

(11) Publication number: **0 196 743**
**A2**

# (12) EUROPEAN PATENT APPLICATION

(21) Application number: **86300616.9**

(22) Date of filing: **29.01.86**

(51) Int. Cl.⁴: **G 01 N 33/52**
G 01 N 31/22, C 07 B 63/00

(30) Priority: **31.01.85 IL 74204**
**11.12.85 IL 77299**

(43) Date of publication of application:
**08.10.86 Bulletin 86/41**

(84) Designated Contracting States:
**AT BE CH DE FR GB IT LI NL SE**

(71) Applicant: **SAVYON DIAGNOSTICS LTD.**
**1 Jabotinsky Street**
**Ramat-Gan(IL)**

(72) Inventor: **Ben-Michael, Abraham**
**8 Rehavat Ilan Street**
**Ramat-Ilan Givat-Shmuel(IL)**

(74) Representative: **Beresford, Keith Denis Lewis et al,**
**R.G.C. Jenkins & Co. 12-15, Fetter Lane**
**London EC4A 1PL(GB)**

## (54) Stable chemical compositions containing chromogenic materials and peroxides, and method for obtaining them.

(57) Chromogenic materials which react to give a color reaction when in the presence of a peroxide ROOR' decomposing to give a radical oxygen are unstable when dissolved to give working solutions for use in chromogenic reactions. Compositions containing in addition to the chromogenic material also a peroxide needed in the said reaction are also unstable, leading to premature appearance of color.

Stable chemical compositions containing chromogenic materials and stable compositions containing mixtures of chromogenic materials and peroxides are described, which can be used in chromogenic reactions after having been stored for long periods of time. Also described are stabilizing chemical compositions and methods for obtaining the said stable compositions.

EP 0 196 743 A2

Croydon Printing Company Ltd.

0196743

TITLE MODIFIED
see front page

# STABLE CHEMICAL COMPOSITIONS CONTAINING CHROMOGENIC MATERIALS OR MIXTURES OF CHROMOGEN MATERIALS AND PEROXIDES, AND STABILIZING CHEMICAL COMPOSITIONS AND METHODS FOR OBTAINING THE STABLE CHEMICAL COMPOSITIONS

## Field of the invention

The present invention relates to methods for obtaining stable chemical compositions containg chromogenic materials, to stabilizing chemical compositions for obtaining stable chemical compositions containing chromogenic materials, to methods for obtaining stable chemical compositions containing mixtures of chromogen materials and peroxides, to stabilizing chemical compositions for obtaining stable chemical compositions containing mixtures of chromogen materials and peroxides, and to stable chemical compositions obtained thereby.

## BACKGROUND OF THE INVENTION

Throughout this specification, a chromogen material is defined as a material which changes its colour when in the presence of a peroxide ROOR' which decomposes to yield oxygen. R and R', as herein defined, may each be independently hydrogen or any organic substituent which can suitably form an organic peroxide or hydroperoxide.

Chromogen materials are of importance in a variety of chemical and clinical tests, and are commonly employed in a wide number of practical applications, e.g., for qualitative or even quantitative tests.

ROOR' causes the change in colour of the chromogen material, via a radical oxygen, when it is decomposed according to the reaction:

$$ROOR' \xrightarrow{Z} ROR' + O^*$$

wherein Z may be any agent which causes this decomposition reaction to take place, for example: UV light, metal ions, active enzymes like peroxidase or catalase, etc., or a pseudo-peroxidase like hemoglobin or Cytochrom-C.

Since, as stated, decomposition of ROOR' takes place easily, and since the colour reaction must take place at the desired time and under controlled conditions, the art has accepted as an unavoidable limitation that chromogen solutions cannot be stored for long periods of time but must be normally prepared afresh before each use. This problem is even more severe when it is desired to prepare chromogen solutions which already contain the desired peroxide. Even when the art has succeeded in obtaining such solutions which can be stored for short periods of time, severe limitations are imposed on the storage thereof or on their use (e.g., use is sometimes forbidden in direct sunlight).

Some examples of solutions involving chromogenic products and use thereof are as follows:

Liem et al (Anal. Biochem. 98, 338-393 (1979)) report the use of 3,3',5,5'-Tetramethylbenzidine dihydrochloride for the quantitative determination of hemoglobin. Hydrogen peroxide is converted to water and oxygen by peroxidase. Tetramethylbenzidine dihydrochloride in water or acetic acid is mixed with a water solution of freshly prepared hydrogen peroxide. This solution, if stored in the refrigerator, can be used for several days.

Levinson and Goldman (Clin. Chem. 28/3. 471-474 (1981)) used a TMB solution in water/acetic acid for measuring hemoglobin in plasma. The working solution containing hydrogen peroxide was stable at room temperature for 4 hours.

Other examples of solutions to be freshly prepared before use, accordinq to the art, are those based on 3-amino-9-ethylcarbazole (Kaplow, L.S., A.J. Chem. Pathol. 63 , 451, 1975), 4-chloro-1-naphtol, for electron microscopic immunoperoxidase staining of insulin (Baskin et al, J. Histochem. and Cytochem., 30 (7) 710-712 (1982)), (A.G.E. Pearse, Histochemistry - Theoretical and Applied, p. 230, vol.1 (1980)).

In a single instance (Diagnostic Procedures for Viral, Rickettsial and Chlamydial Infections (1979), p. 160) a solution of benzidine dihydrochloride is obtained which is stable in the presence of hydhrogen peroxide for approximately 3 months.

There is also a large number of commercially available reagents and kits, employing chromogenic/hydrogen peroxide action, all of which are subject to strict limitations, of which the following are some examples.

Roche manufactures a Beta Specific Monoclonal Enzyme Linked Immunosorbent Assay for Pregnancy. The chromagen reagent employed is tetramethylbenzidine in methanol and the conjugate reagent is peroxidase. The manufacturer directs the user to keep all reagents tightly closed and upright, and not to store reagents in direct sunlight during use. The reagents are mixed immediately before use.

A kit for the Quantitative Immunoenzyme Determination of IgG Antibodies to Rubella Virus in Serum or Plasma Samples, manufactured by Sorin/Biomedica employs two constituents of the reagent: 1) $H_2O_2$ in phosphate-citrate buffer at pH5.15 2) merthiolate and ortho-phenylenediamine·2HCl lyophilized. The reagent obtained by mixture of the above two components must be used within 15-30 min., and should be sheltered from intense light.

In the 1983 DAKOPATTS price list there are found three chromogens for peroxidase staininq: diaminobenzidine (DAB), orthophenylenediamine (oPD) and 3-amino-9-ethylcarbazole (AEC). It is specified that stock solutions of DAB and oPD should be kept at -20$^{o}$C, AEC at 4-8$^{o}$C. Hydrogen peroxide should not be added until shortly before use.

The instructions for preparing HRP color development solution for use in Bio-Rad's Immun-Blot assay system provide for the addition of ice cold $H_2O_2$ immediately prior to use.

The immunoperoxidase staining kit for human antigens, manufactured by Lerner Laboratories, employs 3-amino-9-ethylcarbazole as chromogen. $H_2O_2$ and the reagent are added stepwise during testing.

Although chromogenic reagents are widely used for a large number of applications, the art has not, so far, succeeded in providing a method for obtaining peroxide/chromogen -containing solutions which are stable for long periods of time and which do not require strict storage and handling limitations. Further, even in the absence of a peroxide, chromogen solutions are often unstable. These facts have limited the use and application of chromogenic techiques.

SUMMARY OF THE INVENTION

It is an object of the present invention to provide a method and stabilizing chemical compositions for obtaining stable chemical compositions containing chromogen materials.

It is a further object of the invention to provide a method and stabilizing chemical compositions for obtaining stable chemical compositions containing mixtures of chromogenic materials and peroxides.

It is still another object of the invention to provide stable chemical compositions containing chromogen materials and stable chemical compositions containing mixtures of chromogenic materials and peroxides.

The exact nature of the chromogen is not essential in the present invention, although different chromogens may exhibit stability for different periods of time, all of which, however, exhibit long-term and improved stability when stabilized according to the invention. Examples of fully soluble and partially precipitating chromogens are:

o-dianizidine; o-toluidine; 5-amino salicilic acid; chloronaphthol; benzidine; tetramethylbenzidine; 3-amino-9-ethylcarbazole; dichloronaphthol; dibromonaphthol; 3,3',5,5'- tetramethylbenzidine dihydrochloride; 3,3',5,5'-tetramethylbenzidine (dihydrochloride dihydrate); 3-methylbenzothiazole-2,1-hydrazone; parahydroxyphenyl acetic acid; 2,2'-azino-di (3- ethylbenzothiazoline sulfonic acid); guaiacol; o-tolidine; pyrogalol.

Examples of inorganic and organic peroxides are, e.g., $H_2O_2$, tert-butyl-hydroperoxide 2-butanone peroxide, and Cumene hydroperoxide.

The invention can further be usefully exploited in self-generating systems. By self-generating systems it is meant solutions in which the peroxide needed for the chromogenic reaction is generated "in situ", instead of being externally added as it is done, for example, in the above mentioned kits.

The chemical composition for stabilizing chromogen-containing solutions according to the invention is characterized in that it comprises:

a. an aqueous solvent medium;

b. a water miscible organic solvent;

c. a compound comprising at least two unsaturations and comprising at least one cyclic moiety;

Preferably, the water miscible organic solvent has a gas-phase dipole moment of at least about 1.60 D, and most preferably about 1.69 D.

According to a preferred embodiment of the invention, the aqueous solvent medium is a buffer solution.
In connection with the stabilizing compounds of the present invention, by unsaturations it is meant ethylenic double bonds or aromatic or heteroaromatic unsaturations.

According to a preferred embodiment of the invention, the unsaturations are included in a cyclic moiety. According to this embodiment, therefore, ethylenic unsaturations may not be present and the compound comprising at least two unsaturations and at least one cyclic moiety may be a cyclic compound, e.g., a substituted benzene ring.

According to a preferred embodiment of the invention, the compound comprising at least two unsaturations and at least one cyclic moiety is selected from among:

a compound of the general formula:

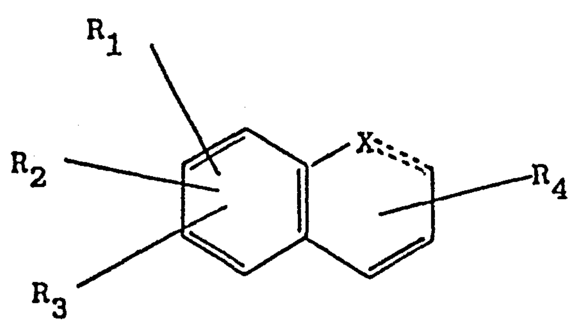

(I)

wherein each of $R_1$, $R_2$, $R_3$ and $R_4$ may be independently hydrogen, hydroxy, $CF_3$, halogen, nitro, $SO_2OH$, $NHCH_2CH_2NH_2$ or lower alkyl and X is nitrogen, oxygen, carbon- or sulphur;

or a compound of formula:

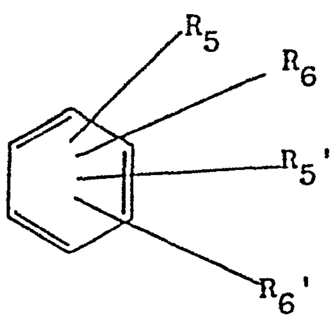

(II)

in which $R_5$, $R_{5'}$, $R_6$ and $R_{6'}$ may each be hydrogen, lower alkenyl, $-NH_2$, phenyl optionally substituted, $-NO_2$, halogen, $-OCH_3$, hydroxy, $COOC_6H_5$, COOH, $CH_2COOH$ or $SO_2OH$;

or a compound of formula

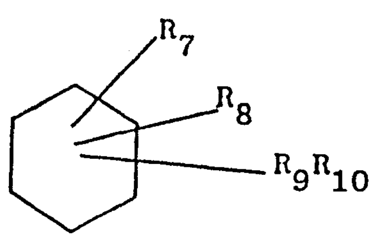

(III)

in which $R_7$ and $R_8$ may each be hydroxy or lower

alkenyl, $R_9$ is alkenyl and $R_{10}$ is an azulene group optionally substituted by one or more straight or branched alkyl group of $C_1$ - $C_{10}$;

or a compound of formula

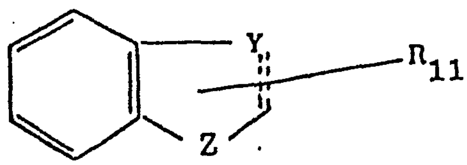

(IV)

wherein Y and Z may each be oxygen, nitrogen- or sulphur and $R_{11}$ is hydrogen or hydroxyphenyl;

or a compound of formula

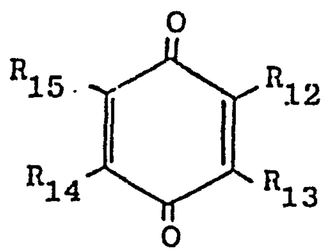

(V)

wherein each of $R_{12}$, $R_{13}$, $R_{14}$ and $R_{15}$ may independently be hydrogen, hydroxy or lower alkyl;

or a salt of one of compounds of formula (I), (II), (III), (IV) or (V);

or an unsaturated nitrogen heterocyclic compound;

The reason for showing certain of the double bonds in broken lines is that they may be absent, for example in formula 1 if X is oxygen.

The aqueous solution medium further preferably contains 0 to about 5000 ppm of chelating agents. The said chelating agents are preferably selected from pyrophosphate, acetanilide, citrate or a derivative of 8-hydroxyquinoline, either alone

or in admixture of two or more of such chelating agents.

A preferred composition according to the invention contains:

0 to about 200 ppm pyrophosphate
0 to about 200 ppm acetanilide
0 to about 1000 ppm citrate
0 to about 2000 ppm 8-hydroxyquinoline or a salt thereof.

The organic solvent is preferably selected from methanol, ethanol, dioxane, dimethylsulfoxide, sulfolane, acetamide, soluted, dimethylsulfone, hexamethylphosphoric triamide, N-methylacetamide, dioxane, dimethylformamide, soluted trioxane, formamide or tetrahydrofuran.

The compound of formula (I) is preferably selected from among 8-hydroxyquinoline, 4-chloro-7-(trifluoromethyl) quinoline, 5-chloro-7-iodo-8-hydroxyquinoline, 5-chloro-8-hydroxyquinoline or a copper or hemi-sulphate salt of 8-hydroxyquinoline. The compound of formula (II) is preferably aniline 2-sulfonic acid, and the compound of formula (III) is preferably Colecalciferol.
The unsaturated nitrogen heterocycle is preferably selected from among the group comprising:
2-amino-4,6-dimethylpyridine, 2-amino-4,6-dimethylpyrimidine, 2-amino-6-methyl pyridine, 2,4,6-collidine, 3-(aminomethyl) pyridine, 2-quinoxalinol, 4-amino-2,6-dimethylpyridine, 5-ethyl-2-methylpyridine, 4-methylpyrimidine, 2,6-lutidin, ethyl nicotinate, 4-piperidinopyridine, 2-aminoethylpyridine, 4-pyridinecarboxylic acid, pyridine-3-sulfonic acid and purine.

When the aqueous solution medium is a buffer solution, the said buffer is selected from among: phosphate buffer (PB), Tris buffer (hydroxymethyl-aminomethane), borate buffer and

glycine buffer.
When it is desired to obtain an acidic solution, the aqueous solution medium is preferably water-soluted citric acid, and when it is desired to obtain a basic solution, a carbonate buffer.

The stable chemical composition according to the invention is characterized in that it comprises:

a. an aqueous solvent medium or a buffer solution;

b. 0 to about 5000 ppm of chelating agents;

c. a water miscible organic solvent preferably having a gas-phase dipole moemnt equal to or greater than 1.60 D, and most preferably greater than 1.69 D;

d. a compound comprising at least two unsaturations and comprising at least one cyclic moiety; and

e. a chromogenic material

The chromogen material is preferably selected from the group comprising:

chloronaphthol, benzidine, tetramethylbenzidine, 3-amino-9-ethylcarbazole, dichloronaphthol, dibromonaphthol, 3,3',5,5' - tetramethylbenzidine dihydrochloride, 3,3',5,5'-tetramethylbenzidine (dihydrochloride dihydrate), 3-methylbenzothiazole -2,1-hydrazone, parahydroxyphenyl acetic acid, 2,2'-azino-di (3-ethylbenzothiazoline) sulfonic acid, 4-aminoantipyrin/alfa-naphthol, o-dianizidine, o-toluidine, 5-amino salicylic acid, guaiacol, o-tolidine, pyrogallol.

The said unsaturations, as hereinbefore defined, may be

ethylenic double bonds or aromatic or heteroaromatic unsaturations. The said compound comprising at least two unsaturations and comprising at least one cyclic moiety may therefore be a cyclic compound, e.g., a substituted benzene ring.

According to a preferred embodiment of the invention, the compound comprising at least two unsaturations and at least one cyclic moiety is selected from among one of the compounds of formula (I), (II), (III), (IV) or (V), or a salt thereof, or an unsaturated nitrogen heterocycle.

The aqueous solution medium may further contain 0 to about 5000 ppm of chelating agents. The said chelating agents are preferably selected from pyrophosphate, acetanilide, citrate, nitrilotriacetic acid, or a derivative of 8-hydroxyquinoline, either alone or in admixture of two or more of such chelating agents.

According to a preferred embodiment of the invention the stable chemical composition contains:

0 to about 200 ppm pyrophosphate
0 to about 200 ppm acetanilide
0 to about 1000 ppm citrate
0 to about 2000 ppm 8-hydroxyquinoline or a salt thereof.

The organic solvent is preferably selected from methanol, ethanol, dioxane, dimethylsuylfoxide, sulfolane, acetamide, soluted dimethylsulfone, hexamethylphosphoric triamide, N-methylacetamide dioxane, dimethylformamide, soluted trioxane, formamide or tetrahydrofuran.

The compound of formula (I) is preferably selected from among 8-hydroxyquinoline, 4-chloro-7-(trifluoromethyl) quinoline,

5-chloro-7-iodo-8-hydroxyquinoline, 5-chloro-8-hydroxyquinoline or a copper or hemi-sulphate salt of 8-hydroxyquinoline. The compound of formula (II) is preferably aniline 2-sulfonic acid, and the compound of formula (III) is preferably Colecalciferol. The unsaturated nitrogen heterocycle is preferably selected from among the group comprising:
2-amino-4,6-dimethylpyridine, 2-amino-4,6-dimethylpyrimidine, 2-amino-6-methyl pyridine, 2,4,6-collidine, 3-(aminomethyl) pyridine, 2-quinoxalinol, 4-amino-2,6-dimethylpyridine, 5-ethyl-2-methylpyridine, 4-methylpyrimidine, 2,6-lutidin, ethyl nicotinate, 4-piperidinopyridine, 2-aminoethylpyridine, 4-pyridinecarboxylic acid, pyridine-3-sulfonic acid and purine.

When the aqueous solution medium is a buffer solution, the said buffer is selected from among: phosphate buffer (PB), Tris buffer (hydroxymethyl-aminomethane), borate buffer and glycine buffer. When it is desired to obtain an acidic solution, the aqueous solvent medium is preferably water-soluted citric acid, and when it is desired to obtain a basic solution, a carbonate buffer.

The stable chemical composition according to the invention may further contain one or more peroxide(s).

According to a preferred embodiment of the invention, the peroxide is hydrogen peroxide.

According to another preferred embodiment of the invention, the peroxide is an organic hydroperoxide, more preferably Cumene hydroperoxide or tert-butylhydroperoxide.

According to still another preferred embodiment of the invention, the peroxide is an organic peroxide, preferably 2-butanone peroxide.

The method for obtaining the stable chemical compositions according to the invention comprises carrying out in any convenient order the steps of:

a. preparing an aqueous solvent medium containing 0 to about 5000 ppm of chelating agents;

b. preparing a chromogen solution in a water-miscible organic solvent, in the presence of a compound comprising at least two unsaturations and comprising at least one cyclic moiety;

c. mixing solutions (a) and (b); and

d. adjusting the pH.

The method for obtaining the stable chemical compositions of the invention, which further contain a peroxide, comprises carrying out in any convenient order the steps of:

a. preparing an aqueous solvent medium containing 0 to about 5000 ppm of chelating agents;

b. preparing a chromogen solution in a water-miscible organic solvent, in the presence of a compound comprising at least two unsaturations and comprising at least one cyclic moiety;

c. mixing solutions (a) and (b);

d. adjusting the pH; and

e. adding the desired peroxide.

It should be noted that, according to the art, peroxides are very sensitive to and easily decomposed by dirt and organic

materials. All the numerous compositions which were prepared according to the invention were therefore tested for stability under "dirt conditions" by the introduction of dust. None of the tested compositions lost stability even under such conditions.

The addition of chelating agents is rendered necessary by the presence of ions such as $Al^{+++}$, $Ni^{++}$, $Mg^{++}$, $Ca^{++}$, $Hg^{+}$ and $Hg^{++}$, which are commonly present either in the water - if not highly purified - or in the glass vessels employed, which ions catalyze the decomposition of the peroxide. In the event, however, that total absence of such ions can be obtained, the chelating agents could be dispensed with.

It should be noted that while the crucial requirements for the organic solvents is its dipole moment, it is readily understood by the person skilled in the art that sufficient solubility of a specific chromogen in a chosen organic solvent is essential, and that chromogen/solvent pairs may exist which are incompatible for practical purposes.

It should be further noted that the pH of the stabilized solution may vary over a wide range, without resulting in a sensible destabilization thereof. The desired pH is mainly dictated by the optimal conditions for the dissolution of the chromogen in the organic solvent and for the activity of the ROOR' decomposing agent.

The aforesaid and other characteristics and advantages of the invention will be better understood through the following illustrative and non-limitative examples.

Example 1

Preparation of stable solution containing

4-chloro-1-naphthol.

1. Preparation of solution A

500 mg 4-chloro-1-naphthol and 1000 mg 8-hydroxyquinoline-hemi-sulphate salt were dissolved in 200 ml dimethylsulfoxide (dipole moment 3.96 D).

2. Preparation of solution B

To 800 ml distilled water there were added 968 mg Tris buffer, 80 mg acetanilide, 80 mg pyrophosphate and 800 mg citrate.

3. Preparation of final solution

Solution A was slowly added to solution B under stirring. The resulting solution was titrated with 10N NaOH until pH 7.6 was obtained. The solution was allowed to cool to about ambient temperature and thereafter 500 microliters $H_2O_2$ were added. 1000 ml solution were obtained. This solution was stable on the desk for 12 months.

Examples 2 through 105

Several compositions according to the invention were prepared and tested for stability in the so-called "shelf-life test". In this test the composition was kept at three different temperatures: 4, 25 and 37$^{\circ}$C. The compositions were kept in brown amber glass bottles with plastic caps. Destabilization was determined by colour development, and activity by colour development on slide in the presence of a peroxidase. The results of these tests are summarized in Table VI.

The various symbols, abreviations and ranges of the materials

used in Table VI are explained in Tables I to V. The said Tables I to V have the purpose of exemplifying several preferred aqueous and organic solvents, chelating agents and compounds containing at least two unsaturations and at least one cyclic moiety (hereinafter referred to as "TuOcm" compounds) it being understood that the said tables are not intended to be limitative.

The following are the detailed meanings of the columns headings in Table VI:

- n - composition number

- Aq. Solv - Aqueous solvent employed

- Chel. - chelating agent(s) employed

- Org. Solv. - organic solvent employed

- Compd. - TuOcm compound employed

- Chr. - chromogen material employed

- pH rg. - range of pH employed

- shelf life - minimal stability of composition (in months) for the appropriate temperature.

As a stable composition it is meant a composition which reacts to give the desired colour reaction.

Tests have been carried our using hydrogen peroxide as the test peroxide, unless otherwise indicated.

Table I : Aqueous Solvents

| Symbol | Solvent | pH Range | Molarity Range |
|---|---|---|---|
| BB | Borate Buffer | 7.5-8.5 | 0.001-0.05M |
| TB | Tris Buffer | 6.8-7.6 | 0.001-0.05M |
| PB | Phosphate Buffer | 6.8-7.6 | 0.001-0.05M |
| AB | Acetate Buffer | 5.0-6.0 | 0.001-0.05M |
| DW | Distilled or Deionized Water | 6.8-7.6<br>5.0-6.0 | - |
| TW | Tap Water | 6.8-7.6<br>5.0-6.0 | - |
| CA | Water soluted citric acid | 2.0-5.0 | 0.001-0.5M |
| CB | Carbonate Buffer | 8.0-11.0 | 0.001-0.05 M |

Table II : Organic Solvents

| Symbol | Solvent |
|---|---|
| DMSO | Dimethylsulfoxide |
| DMSO2 | Dimethylsulfone[b] |
| TMS | Tetramethylensulfone (Sulfolane) |
| DMF | N,N-Dimethylformamide |
| DMAC | N,N-Dimethylacetamide |
| MetOH | Methanol |
| EtOH | Ethanol[c] |
| MAM | N-Methylacetamide |
| ACA | Acetamide |
| DXA | Dioxane |
| TXA | Trioxane[b] |
| FMA | Formamide |
| THF | Tetrahydrofuran |
| MAC | N-Methylacrylamide |

(b) Melted or water soluted

(c) Dipole moment 1.69 D

Table III : Chelating agents

| Symbol | Substance | Concentration range (ppm) |
|---|---|---|
| A | Acetanilide | 0 - 200 |
| P | Sodium pyrophosphate decahydrate | 0 - 200 |
| C | citric acid, tri sodium salt | 0 - 1000 |
| 8HQ | 8-Hydroxyquinoline | 0 - 2000 |
| NAA | Nitrilotriacetic acid | 0 - 200 |

Table IV :[a] TuOcm Compounds

| Symbol | Substance |
|---|---|
| DNS | 2,4-Dinitro-1-naphthol - 7-sulfonic acid |
| DN | 2,4-Dinitro-1-naphthol |
| NE | N-1-naphthyl-ethylenediamine |
| NP | 4-nitro-o-phenylenediamine |
| CI8HQ | 5-chloro-7-iodo-8-hydroxyquinoline |
| C8HQ | 5-chloro-8-hydroxyquinoline |
| CC | colecalciferol (Vitamin $D_3$) |
| DP | 2,4-Dinitrophenol |
| TP | Trinitrophenol |
| NPD | 2-Nitro-p-phenylenediamine |
| COP | 4-chloro-O-phenylenediamine |
| HPB | 2-(2-hydroxyphenyl) benzoxazole |
| HBH | 1-Hydroxybenzothiazole hydrate |
| MP | 2-Methoxyphenol (Guaiacol) |
| 8HQ | 8-hydroxyquinoline |
| 8HQ HS | 8-hydroxyquinoline hemi-sulfate salt |
| THQ | Tetrahydroxy-1,4-quinone |
| CTQ | 4-chloro-7-(Trifluoromethyl) quinoline |
| CTA | 2-(2-chloro-1,1,2-trifluoroethylthio) aniline |
| PT | p-Toluidine |
| OT | o-Toluidine |
| DHN | 1,2-di-hydroxy naphthalene |
| PS | Phenyl-salicylate |
| HABA | 2-(4'-hydroxyazobenzene)benzoic acid |
| AS | aniline 2-sulfonic acid |

Table IV : (Continued)

| Symbol | Substance |
|---|---|
| NQ | 1,4-Naphthoquinone |
| TMB | N,N,N',N'-Tetramethylbenzidine |
| HDPH | o-Hydroxydiphenyl |
| PQD | Primaquine diphosphate |
| QT | Quercetin |
| SDZ | Sodium diatrizoate |
| VAA | Vanillic acid |
| VA | Vanilline |
| THM | Thymol |
| TBP | 2,4,4'-Trihydroxybenzophenone |
| HBP | 2-Hydroxybenzophenone |
| AIQ | 5-Aminoisoquinoline |
| BMA | N-Benzylidenemethylamine |
| BBA | N-Benzylidenebenzylamine |
| BA | 10-Benzylidene-9-anthrone |
| GSA | Guaiacol sulfonic acid |
| AP | 2-Aminopyridine |
| APM | 2-Aminopyrimidine |
| AHHP | 2-Amino-4-hydroxy-6-hydroxypyrazole--[3,4-d]pyrimidine |
| DAA | 4-Dimethylaminoantipyrine |
| DPP | 3,4-Dimethyl-1-phenyl-3-pyrazolin-5-one |
| DPD | 2,2'-Dipyridyl |
| BHA | o-Benzylhydroxylamine.HCl |
| MY | Martius Yellow |
| FA | Flavianic acid |

Table IV : (Continued)

| Symbol | Substance |
|---|---|
| ADP | 2-Amino-4,6-dimethylpyridine |
| ADPM | 2-Amino-4,6-dimethylpyrimidine |
| AMP | 2-Amino-6-methylpyridine |
| AMPM | 2-Amino-6-methylpyrimidine |
| COL | 2,4,6-Collidine |
| 3AMP | 3-(Aminomethyl) pyridine |
| QXO | 2-Quinoxalinol |
| 4ADP | 4-Amino-2,6-dimethylpyridine |
| EMP | 5-Ethyl-2-methylpyridine |
| MPM | 4-methylpyrimidine |
| AEP | 2-Aminoethylpyridine |
| LU | 2,6-Lutidin |
| EN | Ethyl Nicotinate |
| PP | 4-Piperidinopyridine |
| PCX | 4-Pyridine carboxilic acid |
| PSA | Pyridine-3-sulfonic acid |
| PU | Purine |

(a) concentration range: 0 - 2000 ppm

Table V :[a] Chromogens

| Symbol | Substance |
|---|---|
| CN | 4-chloro-1-naphthol |
| DCN | 2,4-Dichloro-1-naphthol |
| BZ | Benzidine.2HCl |
| DMB | 3,3'-Diaminobenzidine.4HCl |
| TMB | 3,3',5,5'-Tetramethylbenzidine |
| TMBd | 3,3',5,5'-Tetramethylbenzidine.2HCl |
| TMBd.2H2O | 3,3',5,5'-Tetramethylbenzidine. $2HCl.2H_2O$ |
| AEC | 3-Amino-9-Ethylcarbazole |
| HPAA | p-Hydroxyphenylacetic acid |
| OTI | o-Tolidine |
| OD | o-Dianisidine |
| G | 2-methoxyphenol (Guaiacol) |
| AAP/N | 4-Aminoantipyrine/alpha-naphthol |
| ABTS | 2,2'-Azino-di-(3-ethylbenzothiazoline sulfonic acid) |
| MTH | 3-Methylbenzo - Thiazol-2,1-hydrazone |
| RT | Rutin |
| 5DAB | 3,5-Diaminobenzoic acid |
| 4DAB | 3,4-Diaminobenzoic acid |
| CT | (+)-Catechin |
| GA | Gallic acid |
| PGA | Propyl gallate |
| PTH | Phenothiazine |

Table V :[a] (Continued)

| Symbol | Substance |
| --- | --- |
| DBA | 4-Dimethylaminobenzoic acid |
| BNP | 1-Bromo-2-naphthol |
| AN | 8-Amino-2-naphthol |
| ACN | 1-Amino-4-chloronaphthol |
| DPA | Diphenylamine |
| 3NPD | 3-Nitro-1,2-phenylenediamine |
| AAP | 4-Aminoantipyrine |
| 4NPD | 4-Nitro-1,2-phenylenediamine |
| ASA | 5-Aminosalycylic acid |
| PGA | Pyrogallol |
| ANS | 8-Amino-1-naphthol-5-sulfonic acid |
| ADPA | p-Aminodiphenylamine |
| AMD | p-Amino-p-methoxy diphenylamine |
| PDA | 1,2-Phenylenediamine |
| pPD | p-Phenylenediamine |
| oPD | o-Phenylenediamine (free base) |
| oPD.HCl | o-Phenylenediamine.HCl |
| TPH | N,N,N',N'-Tetramethyl-p-phenylenediamine.2HCl |
| SGD | Syringaldazine |
| mPD | m-Phenylenediamine |

(a): Concentration range: 0 - 5000 ppm

Table VI : Compositions and Stability Test

| n. | Aq. Solv. | Chel. | Org.Solv. | Cmpd. | Chr. | pH rg. | Shelf life | | |
|---|---|---|---|---|---|---|---|---|---|
| | | | | | | | 4° | 25° | 37° |
| 1 | TB | A+P+C | DMSO | 8HQ HS | CN | 6.8-7.6 | 12 | 6 | 1 |
| 2 | TB | A+P+C | DMSO2 | DMS | G | 6.8-7.6 | 12 | 6 | 1 |
| 3 | TB | A+P+C +8HQ | TMS | DN | OD | 6.8-7.6 | 12 | 6 | 1 |
| 4 | TB | A+P+C | DMF | NE | AAP | 6.8-7.6 | 3 | 1 | 1/2 |
| 5 | TB | A+P+C +8HQ | DMAC | NP | ABTS | 6.8-7.6 | 3 | 1 | 1/2 |
| 6 | PB | A+P+C | DMSO | 8HQ HS | CN | 6.8-7.6 | 12 | 6 | 1 |
| 7 | PB | A+P+NAA +8HQ | DMSO2 | DNS | G | 6.8-7.6 | 12 | 6 | 1 |
| 8 | PB | A+P+C | TMS | DN | OD | 6.8-7.6 | 12 | 6 | 1 |
| 9 | PB | A+P+C | DMF | NE | AAP | 6.8-7.6 | 3 | 1 | 1/2 |
| 10 | PB | A+P+C | DMAC | NP | ABTS | 6.8-7.6 | 3 | 1 | 1/2 |
| 11 | PB | A+P +8HQ | DMSO | 8HQ HS | CN | 6.8-7.6 | 12 | 6 | 1 |
| 12 | PB | A+P+C | DMSO2 | DNS | G | 6.8-7.6 | 12 | 6 | 1 |
| 13 | PB | A+P+C | TMS | DN | OD | 6.8-7.6 | 12 | 6 | 1 |
| 14 | PB | A+P+C | DMF | NE | AAP | 6.8-7.6 | 3 | 1 | 1/2 |
| 15 | PB | A+P+C | DMAC | NP | ABTS | 6.8-7.6 | 3 | 1 | 1/2 |

-25-

Table VI :(Continued)

| n. | Aq. Solv. | Chel. | Org.Solv. | Cmpd. | Chr. | pH rg. | Shelf life 4° | 25° | 37° |
|---|---|---|---|---|---|---|---|---|---|
| 16 | TB | A+P+C | DMSO | 8HQ HS | BZ | 6.8-7.6 | 3 | 1 | 1/2 |
| 17 | TB | A+P+C | DMSO2 | DNS | DMB | 6.8-7.6 | 3 | 1 | 1/2 |
| 18 | AB | A+C +8HQ | TMS | DN | TMB | 5.0-6.0 | 12 | 6 | 1 |
| 19 | AB | A+P+C | DMF | NE | TMBd | 5.0-6.0 | 3 | 1 | 1/2 |
| 20 | AB | A+P+C | DMAC | NP | TMBd .2H2O | 5.0-6.0 | 12 | 6 | 1 |
| 21 | DW | A+P+C | DMSO | 8HQ HS | CN | 6.8-7.6 | 12 | 6 | 1 |
| 22 | DW | P+C +8HQ | DMSO2 | DNS | G | 6.8-7.6 | 12 | 6 | 1 |
| 23 | DW | A+P+C | TMS | DN | OD | 6.8-7.6 | 12 | 6 | 1 |
| 24 | DW | A+P+C | DMF | NE | AAP | 6.8-7.6 | 12 | 6 | 1 |
| 25 | DW | A+P+C | DMAC | NP | TMBd | 5.0-6.0 | 12 | 6 | 1 |
| 26 | TW | A+P+C | DMSO | 8HQ HS | CN | 6.8-7.6 | 12 | 6 | 1 |
| 27 | TW | A+P+C | DMSO2 | DNS | G | 6.8-7.6 | 12 | 6 | 1 |
| 28 | TW | A+P+C | TMS | DN | OD | 6.8-7.6 | 12 | 6 | 1 |
| 29 | TW | A+P+C | DMF | NE | AAP | 6.8-7.6 | 12 | 6 | 1 |
| 30 | TW | A+P+C | DMAC | NP | TMBd | 5.0-6.0 | 12 | 6 | 1 |

-27-

Table VI :(Continued)

| n. | Aq. Solv. | Chel. | Org.Solv. | Cmpd. | Chr. | pH rg. | Shelf life | | |
|---|---|---|---|---|---|---|---|---|---|
| | | | | | | | 4° | 25° | 37° |
| 31 | TB | A+P+C | DMSO | 8HQ HS | ABTS | 6.8-7.6 | 3 | 1 | 1/2 |
| 32 | TB | A+P+NAA | DMSO2 | DNS | CN | 6.8-7.6 | 12 | 6 | 1 |
| 33 | TB | A+P+C | TMS | DN | G | 6.8-7.6 | 12 | 6 | 1 |
| 34 | TB | A+P+C | DMF | NE | OD | 6.8-7.6 | 12 | 6 | 1 |
| 35 | TB | A+P+C | DMAC | NP | AAP | 6.8-7.6 | 12 | 6 | 1 |
| 36 | PB | A+P+C | DMSO | 8HQ HS | ABTS | 6.8-7.6 | 3 | 1 | 1/2 |
| 37 | PB | A+P+C | DMSO2 | DNS | CN | 6.8-7.6 | 12 | 6 | 1 |
| 38 | PB | A+P+C | TMS | DN | G | 6.8-7.6 | 12 | 6 | 1 |
| 39 | PB | A+P+NAA | DMF | NE | OD | 6.8-7.6 | 12 | 6 | 1 |
| 40 | PB | A+P+C | DMAC | NP | AAP | 6.8-7.6 | 12 | 6 | 1 |
| 41 | PB | A+P+C | DMSO | 8HQ HS | ABTS | 6.8-7.6 | 3 | 1 | 1/2 |
| 42 | PB | A+P+C | DMSO2 | DNS | CN | 6.8-7.6 | 12 | 6 | 1 |
| 43 | PB | A+P+C | TMS | DN | G | 6.8-7.6 | 12 | 6 | 1 |
| 44 | PB | A+P+C | DMF | NE | OD | 6.8-7.6 | 12 | 6 | 1 |
| 45 | PB | A+P+C | DMAC | NP | AAP | 6.8-7.6 | 12 | 6 | 1 |

Table VI :(Continued)

| n. | Aq. Solv. | Chel. | Org.Solv. | Cmpd. | Chr. | pH rg. | Shelf life 4° | 25° | 37° |
|---|---|---|---|---|---|---|---|---|---|
| 46 | AB | A+P+C | DMSO | 8HQ HS | TMBd .2H2O | 5.0-6.0 | 12 | 6 | 1 |
| 47 | PB | A+P+C | DMSO2 | DNS | BZ | 6.8-7.6 | 3 | 1 | 1/2 |
| 48 | PB | A+P+C | TMS | DN | DMB | 6.8-7.6 | 3 | 1 | 1/2 |
| 49 | AB | A+P+C | DMF | NE | TMB | 5.6-6.0 | 12 | 6 | 1 |
| 50 | AB | A+P+C | DMAC | NP | TMBd | 5.0-6.0 | 12 | 6 | 1 |
| 51 | DW | A+P+C | DMSO | 8HQ HS | TMDd | 5.0-6.0 | 12 | 6 | 1 |
| 52 | DW | A+P+C | DMSO2 | DNS | CN | 6.8-7.6 | 12 | 6 | 1 |
| 53 | DW | A+P+C | TMS | DN | G | 6.8-7.6 | 12 | 6 | 1 |
| 54 | DW | A+P+C | DMF | NE | OD | 6.8-7.6 | 12 | 6 | 1 |
| 55 | DW | A+P+C | DMAC | NP | AAP | 6.8-7.6 | 12 | 6 | 1 |
| 56 | TW | A+P+C | DMSO | 8HQ HS | TMBd | 5.0-6.0 | 12 | 6 | 1 |
| 57 | TW | A+P+C | DMSO2 | DNS | CN | 6.8-7.6 | 12 | 6 | 1 |
| 58 | TW | A+P+C | TMS | DN | G | 6.8-7.6 | 12 | 6 | 1 |
| 59 | TW | A+P+C | DMF | NE | OD | 6.8-7.6 | 12 | 6 | 1 |
| 60 | TW | A+P+C | DMAC | NP | AAP | 6.8-7.6 | 12 | 6 | 1 |

-29-

Table VI :(Continued)

| n. | Aq. Solv. | Chel. | Org.Solv. | Cmpd. | Chr. | pH rg. | Shelf life 4° | Shelf life 25° | Shelf life 37° |
|---|---|---|---|---|---|---|---|---|---|
| 61 | AB | A+P+C | DMSO | PQD | AAP | 5.0-6.0 | 12 | 6 | 1 |
| 62 | AB | A+P+C | DMSO | HBP | AAP | 5.0-6.0 | 12 | 6 | 1 |
| 63 | AB | A+P+C | DMSO | DPA | AAP | 5.0-6.0 | 12 | 6 | 1 |
| 64 | AB | A+P+C | DMSO | GSA | TMB | 5.0-6.0 | 12 | 6 | 1 |
| 65 | AB | A+P+C | DMSO | VAA | ABTS | 5.0-6.0 | 12 | 6 | 1 |
| 66 | AB | A+P+C | DMSO | GSA | ABTS | 5.0-6.0 | 12 | 6 | 1 |
| 67 | AB | A+P+C | DMSO | RT | ABTS | 5.0-6.0 | 12 | 6 | 1 |
| 68 | AB | A+P+C | DMSO | 8HQ HS | TMB | 5.0-6.0 | 12 | 6 | 1 |
| 69 | AB | A+P+C | DMSO | VAA | TMB | 5.0-6.0 | 12 | 6 | 1 |
| 70 | AB | A+P+C | DMSO | HDPH | TMB | 5.0-6.0 | 12 | 6 | 1 |
| 71 | PB | A+P+C | DMSO | BMA+ BBA+BA | oPD | 6.8-7.6 | 12 | 6 | 1 |
| 72(a) | PB | A+P+C | DMSO | BMA+ BBA+BA | TPH | 6.8-7.6 | 12 | 6 | 1 |
| 73 | PB | A+P+C | DMSO | NE | ASA | 6.8-7.6 | 12 | 6 | 1 |

Table VI :(Continued)

| n. | Aq. Solv. | Chel. | Org.Solv. | Cmpd. | Chr. | pH rg. | Shelf life | | |
|---|---|---|---|---|---|---|---|---|---|
| | | | | | | | 4° | 25° | 37° |
| 74 | PB | A+P+C | DMSO | HPB+ HBH+HBP | oPD | 6.8-7.6 | 12 | 6 | 1 |
| 75 | PB | A+P+C | DMSO | HPB+ HBH+HBP | ASA | 6.8-7.6 | 12 | 6 | 1 |
| 76 | PB | A+P+C | DMSO | HPB+ HBH+HBP | AAP | 6.8-7.6 | 12 | 6 | 1 |
| 77 | PB | A+P+C | DMSO | BMA+ BBA+BA | ASA | 6.8-7.6 | 12 | 6 | 1 |
| 78(a) | PB | A+P+C | DMSO | HPB+ HBH+HBP | TPH | 6.8-7.6 | 12 | 6 | 1 |
| 79 | PB | A+P+C | DMSO | AP+APM+ AHHP+DAA | AEC | 6.8-7.6 | 12 | 6 | 1 |
| 80 | AB | A+P+C | DMSO | AP+APM AHHP+DAA | AEC | 5.0-6.0 | 12 | 6 | 1 |
| 81 | PB | A+P+C | DMF | NE | AEC | 6.8-7.6 | 12 | 6 | 1 |

Table VI :(Continued)

| n. | Aq. Solv. | Chel. | Org.Solv. | Cmpd. | Chr. | pH rg. | Shelf life 4° | 25° | 37° |
|---|---|---|---|---|---|---|---|---|---|
| 82 | PB | A+P+C | DMF | HPB+ HBH+HBP | AEC | 6.8-7.6 | 12 | 6 | 1 |
| 83 | PB | A+P+C | DMF | C8HQ+ DPD | AEC | 6.8-7.6 | 12 | 6 | 1 |
| 84(a) | PB | A+P+C | DMSO | BMA+ BBA+BA | pPDA | 6.8-7.6 | 12 | 6 | 1 |
| 85(a) | PB | A+P+C | DMSO | GSA | SGD | 6.8-7.6 | 12 | 6 | 1 |
| 86(b) | TB | A+P+C+ 8HQ | TMS | DN | OD | 6.8-7.6 | 12 | 6 | 1 |
| 87(b) | BB | A+P+8HQ | DMSO | 8HQ HS | CN | 7.5-8.5 | 12 | 6 | 1 |
| 88(b) | BB | A+P+C | DMAC | NP | ABTS | 7.5-8.5 | 3 | 1 | 1/2 |
| 89(b) | DW | A+P+C | DMF | NE | AAP | 6.8-7.6 | 12 | 6 | 1 |
| 90(b) | TW | A+P+C | DMAC | NP | TMBd | 5.0-6.0 | 12 | 6 | 1 |
| 91(b) | BB | A+P+C | DMSO2 | DNS | CN | 7.5-8.5 | 12 | 6 | 1 |

-31-

Table VI :(Continued)

| n. | Aq. Solv. | Chel. | Org.Solv. | Cmpd. | Chr. | pH rg. | Shelf life 4° | 25° | 37° |
|---|---|---|---|---|---|---|---|---|---|
| 92(b) | PB | A+P+C | DMSO | 8HQ HS | ABTS | 6.8-7.6 | 3 | 1 | 1/2 |
| 93(b) | DW | A+P+C | DMF | NE | OD | 6.8-7.6 | 12 | 6 | 1 |
| 94(a) | TB | A+P+C | DMF | NE | AAP | 6.8-7.6 | 3 | 1 | 1/2 |
| 95(a) | PB | A+P+C | DMSO2 | DNS | G | 6.8-7.6 | 12 | 6 | 1 |
| 96(a) | AB | A+P+C | DMAC | NP | TMBd. 2H2O | 5.0-6.0 | 12 | 6 | 1 |
| 97(a) | PB | A+P+C | DMF | NE | OD | 6.8-7.6 | 12 | 6 | 1 |
| 98(a) | TW | A+P+C | DMSO2 | DNS | CN | 6.8-7.6 | 12 | 6 | 1 |
| 99(a) | PB | A+P+C | DMF | HPB+ HBH+HBP | AEC | 6.8-7.6 | 12 | 6 | 1 |
| 100 | PB | A+P+C | DMSO | 8HQ HS | TMB+ CN | 6.8-7.6 | 12 | 6 | 1 |
| 101 | AB | A+C+ 8HQ | TMS | DN | TMB+ ASA | 5.0-6.0 | 12 | 6 | 1 |

Table VI :(Continued)

| n. | Aq. Solv. | Chel. | Org.Solv. | Cmpd. | Chr. | pH rg. | Shelf life | | |
|---|---|---|---|---|---|---|---|---|---|
| | | | | | | | 4° | 25° | 37° |
| 102 | PB | A+P+C | DMSO | NE | ASA+ AAP | 6.8-7.6 | 12 | 6 | 1 |
| 103 | AB | A+P+C | DMSO | DPA | AAP+ CN | 5.0-6.0 | 12 | 6 | 1 |
| 104 | PB | A+P+C | DMSO | NE | ASA+ CN | 6.8-7.6 | 12 | 6 | 1 |

---

Peroxide: (a) Cumene Hydroperoxide
(b) Absence of peroxide

Example 106

Preparation of stable solution containing o-Dianisidine:

a) 50 mg citric acid tri-sodium salt, 5 mg acetanilide and 5 mg sodium pyrophosphate decahydrate were disolved in 80 ml Tris Buffer 0.001M, having a pH of 7.2.

b) 10 mg 8-hydroxyquinoline, 100 mg 2,3-dinitro-1-naphthol-7 sulfonic acid and 20 mg o-dianisidine were dissolved in 20 ml sulfolane.

Solutions a) and b) were mixed and titrated to obtain a pH 7.2. 10 Microliters $H_2O_2$ were added to the resulting solution.

The solution had a shelf-life of 12 months at 4°C, 6 months at 25°C and 1 month at 37°C.

Example 107

Preparation of stable solution containing 4-Aminoantipyrine/alpha-naphthol.

a) 50 mg citric acid tri-sodium salt, 5 mg acetanilide and 5 mg sodium pyrophosphate decahydrate were added to 75 ml Phosphate Buffer 0.001M having pH 7.6.

b) 100 mg 8-hydroxyquinoline hemi-sulfate salt, 50 mg 4-aminoantipyrine and 500 mg alpha-naphthol were dissolved in 25 ml N,N-Dimethylacetamide.

Solutions a) and b) were mixed and NaOH 0.1N was added, to obtain a pH 6.9, together with 10 microliters $H_2O_2$.
The shelf-life of the resulting solution was as in Example 62.

Example 108

Preparation of stable solution containing 4-Aminoantipyrine/phenol.

Example 63 was repeated but using 500 mg phenol instead of alpha-naphthol. The results were as in Example 63.

Example 109

Preparation of stable solution containing 2,4-dichloro-1-naphthol

a) 40 mg citric acid tri-sodium salt, 8 mg acetanilide and 8 mg sodium pyrophosphate decahydrate were dissolved in 50 ml distilled water.

b) 50 mg 2,4-dinitro-1-naphthol and 25 mg 3,3',5,5'-tetramethylbenzidine.2HCl.2$H_2O$ were dissolved in 50 ml dimethylsulfone, soluted in DW.

Solutions a) and b) were mixed and brought to a pH 5.0. 15 microliters $H_2O_2$ were added. The stability results obtained were as in the previous example.

Example 110

a) 40 mg citric acid tri-sodium salt, 4 mg acetanilide and 4 mg sodium pyrophosphate were dissolved in 60 ml tap water.

b) 10 mg 8-hydroxyquinoline, 50 mg guaiacol and 100 mg 8-hydroxyquinoline hemi-sulfate salt were dissolved in 40 ml DMSO.

Compositions a) and b) were mixed together and with 10

microliter $H_2O_2$. The pH of the resulting composition was brought to 7.0. Shelf lives of not less than 12,6 and 1 months for 4, 25 and 37°C respectively were obtained.

Example 111

a) 7 mg acetanilide, 5 mg sodium pyrophosphate and 15 mg citric acid tri-sodium salt were dissolved in 90 ml phosphate buffer.

b) 20 g trioxane were completely dissolved in the above solution.

c) 100 mg chloronaphthol and 200 mg 8-hydroxyquinoline were then dissolved in the above solution which was then titrated to obtain a pH7.2-7.4.

d) 100 microliters $H_2O_2$ (30% aqueous solution) were then added.

Shelf lives obtained as in the preceding example.

Example 112

Preparation of 100 ml stable solution.

A solution is prepared which contains 100 mg amino-ethylcarbazole in 30 ml DMF, 100 mg 2-amino-4-picoline, acetate buffer (0.01M, pH 5.0, 70 ml), containing sodium pyrophosphate (100 mg), sodium citrate (100 mg) and acetanilide (100 mg), and 30 microliter $H_2O_2$. The solution obtained shows a stability of 1 month at 37°C, 6 months at 25°C and 12 months at 4°C.

Example 113

The solution is prepared as above, but using 100 microliter 2-butanone peroxide instead of $H_2O_2$. Shelf lives obtain as in Example 112.

Example 114

A solution is prepared containing 100 mg o-phenylenediamine in 30 ml DMSO, 100 mg 2-amino-4-picoline, phosphate buffer (0.01M, pH 7.2, 70 ml) containing 100 mg sodium pyrphosphate, 100 mg sodium citrate and 100 mg acetanilide, and 100 microliter $H_2O_2$. The shelf life of the obtained solution is 3 weeks at 37 °C, 2 months at 25° C and 12 months at 4° C.

Example 115

A solution is prepared containing 100 mg tetramethylbenzidine in 40 ml DMSO, 1 g 2,4,6-collidine, acetate buffer (60ml, 0.01M, pH 4.0) containing 50 mg sodium pyrophosphate, 50 mg sodium citrate and 50 mg acetanilide, and 100 microliter 2-butanone peroxide. Shelf lives obtain as in Example 112.

Examples 116 and 117

Example 115 is repeated using 100 microliter cumene hydroperoxide and 100 microliter tert-butylhydroperoxide as the peroxides. Shelf lives obtain in each case as in Example 112.

Example 118

A solution of 100 mg 4-chloro-1-naphtol in 30 ml DMSO is prepared, to which is added 1 g guaiacol sulfonic acid, phosphate buffer (70 ml, 0.05M, pH 7.4) containing 50 mg sodium pyrophosphate, 50 mg sodium citrate and 50 mg

acetanilide, and 50 microliter $H_2O_2$. Shelf life is 1 month at 37°C, 6 months at 25°C and 12 months at 4°C.

Example 119

Example 118 is repeated but using 2,6-lutidin instead of guaiacol sulfonic acid. Shelf lives obtain as in Example 118.

Example 120

A solution is prepared using 100 mg tetramethylbenzydine as the chromogen material in 30 ml DMSO and in 10 g citric acid (soluted in 70 ml distilled water) as the solvent. There are added 60 mg sodium pyrosphosphate, 60 mg acetanilide, 60 mg sodium citrate, 60 mg 8-hydroxyquinoline and 100 microliter Cumene hydroperoxide. The resulting pH of the solution is 2. Shelf lives are 1 month at 37°C, 6 months at 25°C and 12 months at 4°C.

Example 121

4-Chloro-1-naphtol (100 mg in 30 ml DMSO) is soluted in a carbonate buffer (70 ml, 0.02M, pH 9.0). Sodium pyrophosphate (50 mg), acetanilide (50 mg), sodium citrate (50 mg) and 8-hydroxyquinoline (50 mg) are added, and further it is added 50 microliter $H_2O_2$. Shelf lives obtain as in Example 120.

The above examples have been given for the purpose of illustration and are not intended to be limitative. Many variations can be provided in the various chemical compounds, compositions and methods described, without exceeding the scope of the invention.

CLAIMS:

1. A chemical composition for stabilizing chromogen-containing solutions, characterized in that it comprises:

a. an aqueous solvent medium;

b. a water miscible organic solvent;

c. a compound comprising at least two unsaturations and comprising at least one cyclic moiety;

2. A stable chemical composition according to claim 1, characterized in that it further comprises a chromogenic material.

3. The chemical composition of claim 1 or 2, wherein said water miscible organic solvent has a gas-phase dipole moment of at least about 1.60 D and most preferably of at least about 1.69 D.

4. A composition according to claim 1 or 2, characterized in that the aqueous solvent medium is selected from among: phosphate buffer (PB), Tris buffer (hydroxymethyl-aminomethane), borate buffer, glycine buffer, carbonate buffer and water-soluted acetic acid.

5. A composition according to any one of the preceding claims, characterized in that the unsaturations comprise ethylenic double bonds.

›

6. A composition according to claims 1 to 4, characterized in that the unsaturations are aromatic or heteroaromatic unsaturations.

7. A composition according to claim 1 to 4, characterized in that the unsaturations are included in a cyclic moiety.

8. A composition according to any one of the preceding claims, characterized in that the ciclic moiety comprises and unsaturated nitrogen heterocycle.

9. Composition according to any one of the preceding claims, characterized in that the compound comprising at least two unsaturations and comprising at least one cyclic moiety is selected from among:

a compound of the general formula:

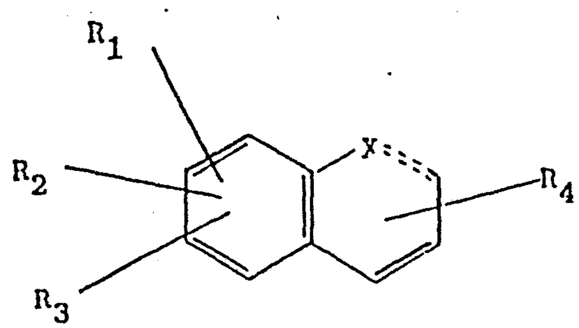

(I)

wherein each of $R_1$, $R_2$, $R_3$ and $R_4$ may be independently hydrogen, hydroxy, $CF_3$, halogen, nitro, $SO_2OH$, $NHCH_2CH_2NH_2$ or lower alkyl and X is nitrogen, oxygen, carbon- or sulphur;

or a compound of formula

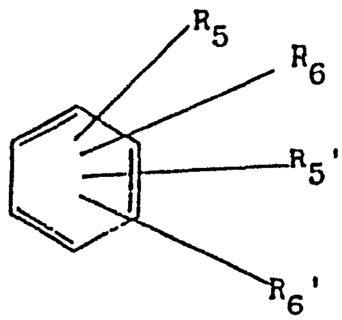

(II)

in which $R_5$, $R_{5'}$, $R_6$ and $R_{6'}$ may each be hydrogen, lower alkenyl, $-NH_2$, phenyl optionally substituted, $-NO_2$, halogen, $-OCH_3$, hydroxy, $COOC_6H_5$, COOH, $CH_2COOH$ or $SO_2OH$;

or a compound of formula

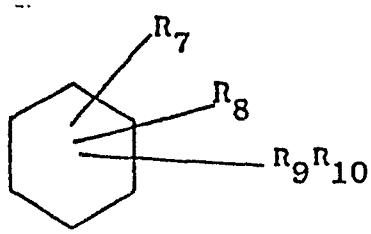

(III)

in which $R_7$ and $R_8$ may each be hydroxy or lower alkenyl, $R_9$ is alkenyl and $R_{10}$ is an azulene group optionally substituted by one or more straight or branched alkyl group of $C_1$ - $C_{10}$;

or a compound of formula

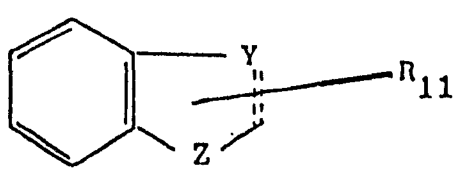

(IV)

wherein Y and Z may each be oxygen, nitrogen- or sulfur and $R_{11}$ is hydrogen or hydroxyphenyl;

or a compound of formula

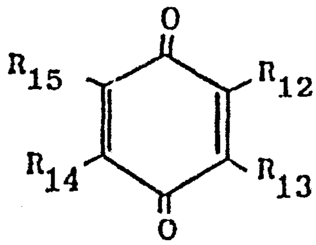

(V)

wherein each of $R_{12}$, $R_{13}$, $R_{14}$ and $R_{15}$ may independently be hydrogen, hydroxy or lower alkyl;

or a salt of one of compounds of formula (I), (II), (III), (IV) or (V);

or an unsaturated nitrogen heterocyclic compound.

10. A composition according to the preceding claims, characterized in that the aqueous solvent medium further contains 0 to about 5000 ppm of chelating agents, the said chelating agents being preferably selected from among pyrophosphate, acetanilide, nitrilotriacetic acid, citrate or a derivative of 8-hydroxyquinoline, either alone or in admixture of two or more of such chelating agents, and more preferably the said aqueous solvent medium contains:

0 to about 200 ppm pyrophosphate
0 to about 200 ppm acetanilide
0 to about 1000 ppm citrate
0 to about 50 ppm 8-hydroxyquinoline or a salt thereof.

11. A composition according to any one of the preceding claims characterized in that the organic solvent is selected from methanol, ethanol, dioxane, dimethylsulfoxide, sulfolane, acetamide, soluted dimethylsulfone,

hexamethylphosphoric triamide, N-methylacetamide, dioxane, dimethylformamide, soluted trioxane, formamide or tetrahydrofuran.

12. A composition according to any one of the preceding claims, characterized in that the compound of formula (I) is selected from among 8-hydroxyquinoline, 4-chloro-7-(trifluoromethyl) quinoline, 5-chloro-7-iodo-8-hydroxyquinoline, 5-chloro-8-hydroxyquinoline or a copper or hemi-sulphate salt of 8-hydroxyquinoline.

13. A composition according to any one of the preceding claims, characterized in that the compound of formula (II) is aniline 2-sulfonic acid.

14. A composition according to any one of the preceding claims, characterized in that the compound of formula (III) is Colecalciferol.

15. A composition according to any one of the preceding claims, in which the unsaturated nitrogen heterocycle is selected from among the group comprising: 2-amino-4,6-dimethylpyridine, 2-amino-4,6-dimethylpyrimidine, 2-amino-6-methyl pyridine, 2,4,6-collidine, 3-(aminomethyl) pyridine, 2-quinoxalinol, 4-amino-2,6-dimethylpyridine, 5-ethyl-2-methylpyridine, 4-methylpyrimidine, 2,6-lutidin, ethyl nicotinate, 4-piperidinopyridine, 2-aminoethylpyridine, 4-pyridinecarboxylic acid, pyridine-3-sulfonic acid and purine.

16. A composition according to claim 15, characterized in that the chromogen material is selected from the group comprising:

chloronaphthol, benzidine, tetramethylbenzidine, 3-amino-9-ethylcarbazole, dichloronaphthol, dibromonaphthol, 3,3',5,5' - tetramethylbenzidine dihydrochloride, 3,3',5,5'-tetramethylbenzidine (dihydrochloride dihydrate), 3-methylbenzothiazole -2,1-hydrazone, parahydroxyphenyl acetic acid, 2,2'-azino-di (3-ethylbenzothiazoline) sulfonic acid, 4-aminoantipyrin/alpha-naphthol, o-dianizidine, o-toluidine, 5-amino salicilic acid, guaiacol, o-tolidine, pyrogallol.

17. A stable chemical composition according to any one of claims 1 to 16, characterized in that it further contains one or more peroxide(s), preferably selected from among hydrogen peroxide, Cumene hydroperoxide, tert-butylhydroperoxide or 2-butanone peroxide.

18. A method for obtaining the stable chemical compositions of the preceding claims, comprising carrying out in any convenient order the steps of:

a. preparing an aqueous solvent medium containing 0 to about 5000 ppm of chelating agents;

b. preparing a chromogen solution in a water-miscible organic solvent, in the presence of a compound comprising at least two unsaturations and comprising at least one cyclic moiety;

c. mixing solutions (a) and (b);

d. optionally adding a peroxide; and

e. adjusting the pH.